# EUROPEAN PATENT APPLICATION

(11) **EP 1 050 305 A1**
(43) Date of publication of application: **08.11.2000**
(21) Application number: 99500069.2
(22) Date of filing: 03.05.1999
(51) Int. Cl.: A61K 35/78, A61P 1/04

(54) **A composition for the treatment of stomach ulcers, duodenal ulcers and ulcers of the digestive system**

(71) Applicant: Gil Garcia, Josefa, C.P. 03007 Alicante (ES)
(72) Inventor: Gil Garcia, Josefa, C.P. 03007 Alicante (ES)

(57) **Abstract**

A composition for treating stomach ulcers, duodenal ulcers and ulcers of the digestive system in general. To produce the composi 5 kilos of CISTUS LADANIFERS (the rock-rose plant) is boiled in 40 litres of distilled water for 45 minutes. The liquid is allowed to cool. Potassium sorbus and liquorice extract are added. Finally the mixture is filtered and sealed into capsules.

## Description

The objective of this invention is a composition for the treatment of stomach ulcers, duodenal ulcers and of the digestive system in general.

There do exist varions well-known products which are used for this purpose. However the secondary effects produced have a negative effect on the patients general health. The composition of this invention is simple and is well accepted by the human body. No complications nor secondary effects have been noted. This implies that this product has a great advantage over other well-known treatments for this problem. The composition is based on the plant called CISTUS LADANIFERS, a member of the CISTACEAS family commonly known as the rock-rose.

The product contains a liquorice extract, potassium sorbus, distilled water dessicated rock-rose (Cistus Ladanifers) which includes the leaves, the stems and the flowers of this plant and in your case any flavour.

First of all, 40 litres of distilled water and 5 kilos of the leaves, stems and flowers of the rock-rose are put into non-rust, steel container which is heated to 100ºC for 45 minutes, stirring all the time. In this way, the resin is extracted from the plant and any posible contamination is eliminated. The liquid is then allowed to cool. Potassium sorbus and the liquid extract are added, and in your case any favour. An anti-foaming agent may be added but only a maximum of 1 gramme per litre. The mixture is then filtered to remove solid particles and residues which form the impurities. The liquid obtained is hermetically sealed into capsules.

## Claims

1. Composition for the treatment of stomach, duodenal and ulcers of the digestive system in general whose exceptional characteristic is the utilisation of 5 kilos of CISTUS LADANIFERS or rock-rose plant boiled in 40 litres distilled water.

2. Composition according to claim 1 whose exceptional characteric is because the ROCK-ROSE plant in mixed in distilled water, when cold, liquorice extract, potassium sorbus, and in your case any flavour are added. The resulting composition is filtered and hermetically sealed in capsules.
